# EUROPEAN PATENT SPECIFICATION

(11) **EP 3 235 536 B1**
(45) Date of publication and mention of the grant of the patent: **06.03.2019**
(21) Application number: 17167633.1
(22) Date of filing: 21.04.2017
(51) Int. Cl.: A61M 25/09

(54) **GUIDEWIRE TORQUER**
FÜHRUNGSDRAHTDREHMOMENTERZEUGER
DISPOSITIF D'ACCOUPLEMENT DE FIL-GUIDE

(30) Priority: 21.04.2016 WO PCT/NL2016/050281
(43) Date of publication of application: 25.10.2017
(73) Proprietor: Marsman, Johan Willem Pieter, 1213 TM Hilversum (NL)
(72) Inventor: Marsman, Johan Willem Pieter, 1213 TM Hilversum (NL)
(74) Representative: V.O.

(56) References cited:
- US-A1- 2005 070 820
- US-A1- 2005 240 120
- US-A1- 2010 100 103
- US-A1- 2012 172 845
- US-A1- 2012 216 385

## Description

### FIELD AND BACKGROUND OF THE INVENTION

The present invention relates to a guidewire torquer.

A guidewire torquer is a device that is used in a number of different medical procedures to guide vascular catheters, catheter-mounted heart valves, aortic endografts, endotracheal tubes, or gastric feeding tubes and the like into a patient towards a desired location within the patient. Guidewires are used in a number of diagnostic and interventional fields, such as interventional cardiology, diagnostic and interventional radiology, vascular surgery, minimally invasive vascular interventions such as angioplasty, stenting, thrombolysis, transcatheter aortic valve insertion (TAVI), and endovascular abdominal aortic aneurysm repair (EVAR).

In vascular uses, a physician is required to navigate the guidewire through the vasculature of the patient. This is done in order to position the distal end of the guidewire at the desired location. Then a diagnostic or therapeutic catheter is fed over the guidewire to the desired location for the planned vascular intervention. In the text, the distal end of the guidewire is the end that is to enter the human body. The proximal end of the guidewire is in the hands of the physician and is not inserted into the body.

The distal end of the guidewire generally has an angled tip adjusted to help steer the guidewire. Positioning the distal end of the guidewire at the desired location can be tough and time consuming due to complex vascular anatomy and due to abnormalities of the vessel lumen caused by vascular disease. The physician manipulates the distal end of the guidewire through the vasculature of the patient to the desired location by pinching and torqueing the proximal end of the guidewire with his fingers.

Guidewires are relatively fine and difficult to grip between the physician's fingers, thereby making the positioning of the guidewire challenging. Handling of the guidewire is hampered by the fact that the guidewire is often slippery particularly when wetted with saline or blood. A guidewire is also intended to be smooth by means of various kinds of coating in order to provide lubricity between the guidewire surface and the inner surface of the vessel wall.

Due to the slipperiness of the guidewire the physician cannot accurately and securely rotate or move the guidewire lengthwise in and out of the body. It is also difficult to reliably feel with his fingers to what extent the guidewire follows his steering manipulations.

As such, a device called a guidewire torque device, guidewire torquer, or steering handle, is often affixed to the guidewire in order to allow the physician to better grip and impart motion to the guidewire. That is, the guidewire torquer device is intended to allow the physician to securely control the movements of the guidewire and to steer the distal end of the guidewire by rotational and longitudinal manipulation of the guidewire.

One disadvantage of prior art guidewire torquers is that they are configured to be attached from and over the proximal end of a guidewire. Generally, guidewire torquers must be back loaded over the proximal end of the guidewire, and then advanced along the guidewire until a suitable location is reached. After that a reliable fixation between guidewire and torquer is required, which may be unreliable or damage the guidewire by kinking or breakage or detachment of surface particles.

Furthermore, prior art guidewire torquers are complex, consisting of multiple moving parts, and therefore are relatively expensive.

For instance U.S. patent application publications US 2012/172845, US 2010/100103 and US 2005/070820 disclose torque devices in which a guidewire can be fixated by clamping the guidewire into a non linear configuration by moving parts of a torque device body relative to each other and subsequently securing the parts to each other. U.S. patent application publication US 2005/240120 discloses a torque device in which a guidewire can be fixated in a non linear configuration by rotating first and second body parts relative to a central cam part so that slits in the parts become misaligned to each other.

U.S. patent application publication US 2012/0216385 discloses a retaining device for retaining one or more medical lines to a surface. For example, the device can securely retain a proximal end of a guidewire extending out of a patient from an entry point to the medical surface and ensure that the proximal end of the guidewire does not become contaminated or tangled or confused with other wires in the procedural area. The retaining device has a body of a deformable, flexible and compressible material such as a polymer elastomer or polymer foam that compresses around the line and produces increased surface contact allowing for greater retention. An attachment mechanism is coupled to the flexible body of the device for securing the flexible body to a medical surface, thereby securing one or more medical lines to the medical surface. The flexible body has at least one non linear slit, slot or thin cut formed in the top surface and extending to a depth greater than the diameter of the medical line to be retained. The slits may have a tapered configuration, e.g. being wider at one end compared to the other end. In one example, the body has sinusoidal shaped slits in the top surface and extending towards the bottom surface so that slit depth (from the top surface) is greater than the diameter of the medical device. The slits are thin cuts made into the body. Further slots communicate with and are perpendicular with the slits. Alternately, the further slots could be slits or round orifices.

### SUMMARY OF THE INVENTION

In order to improve such prior art, the present invention provides a guidewire torquer according to claim 1.

A main advantage of such a guidewire torquer is that a superior and reliable manual control of the guidewire can be provided while the mounting of the guidewire torquer to the guidewire can be performed in a simple manner.

Also the mounting of the guidewire torquer to the guidewire can be performed at any spot of the guidewire as seen lengthwise to the guidewire, without the need for back loading the guidewire torquer over the proximal end of the guidewire. Because of the frictional fixation of the guidewire inside the guidewire torquer, forces exerted by the guidewire torquer on the guidewire are spread over the contact area of frictional fixation. Therefore, the fixation is highly reliable. Also no movable parts are required to provide such friction.

Due to the fact, that forces exerted by the guidewire torquer on the guidewire are spread over the contact area, the following known problem of the prior art is prevented. In prior art devices clamping elements such as screws or claws pose a risk of detachment of particles from the coating of the guidewire, which is especially so in case of slippage of the guidewire relative to the torquer. Such damage, also known as local stripping of surface material or coating from the guidewire may pose health risks to patients being operated on, when such particles reach the inside of the human body.

Another aspect of possible damage by prior art devices to the guidewire is that a point force exerted by the guidewire torquer leads to deformation of the contour and shape of the guidewire, which could even lead to kinking or breakage of the wire. Such damages are prevented with the present guidewire torquer.

Also, the presented guidewire torquer provides a reliably visible and/or tactile feedback to the physician concerning the rotational orientation of the guidewire within the guidewire torquer, and a visible feedback concerning the insertion depth of the guidewire. According to the prior art such feedback to the physician is missing as the technical development of those devices has been directed solely at the capability to attach the device to the guidewire.

Since the guidewire holding surfaces are part of a single, rigid body portion, the guidewire can be inserted easily and is rigidly fixed against rotation relative to the torquer. Exerting a torque to the guidewire results in very little or no rotation of the guidewire relative to the torquer where the guidewire is engaged by the torquer.

A further aspect according to the present invention provides a kit according to claim 10.

### BRIEF DESCRIPTION OF THE DRAWINGS

While the appended claims set forth the features of the present invention with particularity, further advantages, features and details of the present invention will be elucidated on the basis of a description of one or more embodiments with reference to the accompanying figures, of which:
Fig. 1 provides a perspective view of a first preferred embodiment according to the present invention.
Figs. 2A, 2B, 2C and 2D provide four further views of the embodiment of figure 1.
Figs. 3A, 3B, 3C and 3D provide a further preferred embodiment according to the present invention being mounted on a guidewire.
Figs. 4A and 4B provide two front views of a further preferred embodiment according to the present invention.
Fig. 5 provides a perspective view of a further preferred embodiment according to the present invention.

### DETAILED DESCRIPTION OF THE DRAWINGS

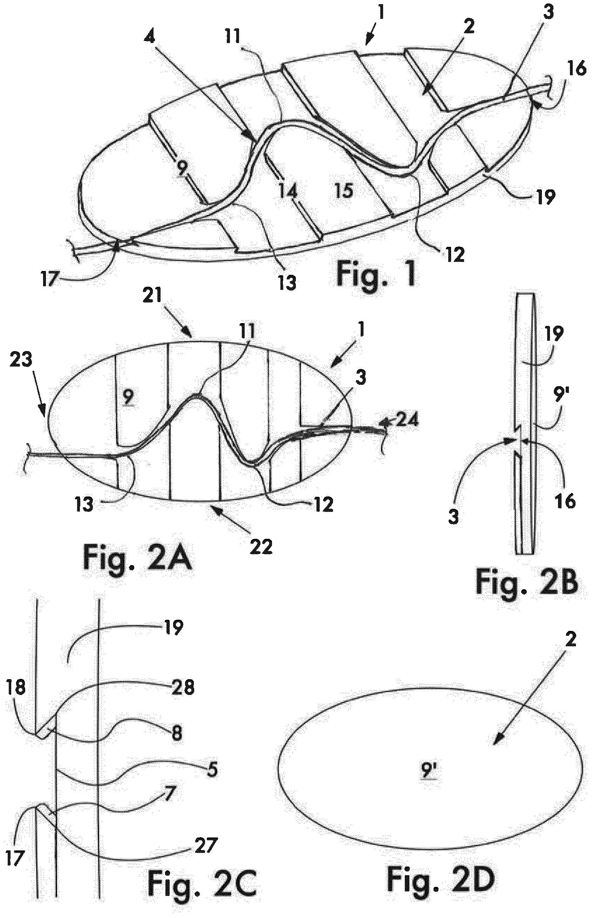

Fig. 1 shows a perspective view of a guidewire torquer 1 according to a first preferred embodiment according to the invention. Figures 2A-2D show further views of the guidewire torquer 1. The guidewire torquer is comprised of a single handling body 2, which may be for instance be (injection) molded, machined or manufactured using an additive process such as 3D-printing. The forming of the guidewire torquer from a single part provides a low complexity and expense of the device. The guidewire torquer 1 has the shape of a substantially flat, oval disc. The longitudinal diameter of the oval disc is about twice as long as the transverse diameter of the oval disc. The guidewire torquer 1 includes a front surface 9, a back surface 9', a top side 21, a bottom side 22, a left side 23, a right side 24 and a circumference surface 19.

The guidewire torquer has 1 a plurality of guidewire holding surfaces 7, 8 oriented for engaging the guidewire from mutually opposite sides of the guidewire and holding the guidewire in an elastically tensioned, bent guidewire shape 3 providing frictional fixation of the guidewire relative to the handling body 2. The guidewire holding surfaces 7, 8 project from an abutment plane defined by an abutment surface 5. A guidewire trajectory of the bent guidewire shape 3 extends along and touches the guidewire holding surfaces 7, 8 and is accessible from a direction in which the abutment surface 5 is facing. The guidewire holding surfaces 7, 8 lean over towards the abutment plane 5 for holding the guidewire adjacent the abutment plane as a result of the tendency of the guidewire to elastically flex back to its original straight shape. The guidewire holding surfaces 7, 8 are part of a single, rigid body portion 2.

The rigid body portion 2 holding the guidewire constitutes a particularly stiff engagement of the guidewire, so that steering can be carried out with great precision and a very direct feel. The handling body 2 is of a material having a modulus of elasticity (Young's tensile at 20 °C) of at least 0.5 GPa and preferably at least 1.0 GPa or at least 1.5 Gpa. For precision and direct feel, the handling body preferably has a material thickness at the abutment surface of at least 2 mm and preferably at least 3 mm. The handling body may for instance be made from a rigid polymer material, such as PET, PLA, ABS, PMMA, POM or PA.

For a direct steering feeling and low manufacturing costs, it is advantageous that the guidewire torquer 1 is made of one part. Because the guidewire torquer is free of handling body portions opposite of each of the guidewire holding surfaces, room is left for slides of a mold defining the overhanging guidewire holding surfaces. If openings are provided in the abutment surface directly adjacent the overhanging guidewire holding surfaces, the overhanging guidewire holding surfaces may be formed by mold portions projecting through these openings in mold opening direction after the handling body has been molded. When manufacturing is carried out by 3D printing, non-releasing shapes, such as overhanging guidewire holding surfaces can be made particularly easily.

For holding the guidewire with sufficient pre-tension to allow a large torque to be exerted, the guidewire holding surfaces are preferably arranged such that, in a view of said bent guidewire shape perpendicular to a plane in which the guidewire is curved, the amplitude of a succession of bends is more than 0.1 and preferably more than 0.2 times the distance between successive peaks or successive deepest positions of valleys in the succession of bends. Also for exerting a large torque it can be provided that at least portions of the guidewire holding surfaces have a higher coefficient of friction relative to a guidewire than other surface portions of the torquer.

For allowing a large torque to be exerted, without having to bend the guidewire beyond its maximum elastic deformability, it is furthermore advantageous if the guidewire holding surfaces are arranged such that, in a view of said bent guidewire shape perpendicular to a plane in which the guidewire 3 is curved, the succession of bends constitutes at least two peaks or, as in the present example, at least two valleys 12.

The guidewire holding surfaces 7, 8 and the abutment surface 5 are shown in greater detail in figure 2C. The abutment surface 5 of the insertion channel is bounded by guidewire holding surfaces 7, 8. The abutment surface 5 is connected with the guidewire holding surfaces 7, 8 via the angular transitions 27, 28. The guidewire holding surfaces 7, 8 extend to free edges 17, 18. Since the guidewire holding surfaces 7, 8 lean over towards a plane defined by the abutment surface 5, the guidewire holding surfaces 7, 8 urge the guidewire towards the abutment surface 5 as a result of the urging force of the guidewire which is inclined to self-straighten.

For holding the guidewire particularly reliably against or near the abutment plane, at least two of the guidewire holding surfaces lean over obliquely towards the abutment surface at an angle of more than 25° and preferably more than 30° or more than 35° (each relative to a direction perpendicular to the abutment plane).

The trajectory along which bent guidewire held in the torquer 1 extends comprises a curved section 4 having one top bend 11 and two bottom bends 12 and 13. The guidewire is forced to follow the bends imposed by the guidewire holding surfaces 7, 8 resulting in normal forces and accordingly friction between the guidewire 3 and the guidewire holding surfaces 7, 8. Because of the friction and the rigid construction of the handling body 2, the guidewire 3 is firmly fixed relative to the handling body 2. For the purpose of operation, the guidewire 3 is arranged against the abutment surface 5 while flexing it past the guidewire holding surfaces 7, 8. Subsequently, the physician is able to control the rotational orientation of the guidewire 3 and a tip at the distal end thereof. So the physician is able to steer and to navigate the guidewire through a vasculature of the patient. The physician is also able to both easily mount the guidewire torquer to the guidewire and to easily unlock the torquer from the guidewire. Therefore the user is able to quickly move the guidewire torquer from the one to the other location on the guidewire. These features are particularly useful by saving time for performing a procedure.

For holding different types of guidewires, the torquer may be provided with at least two groups of guidewire holding surfaces, the groups being arranged for holding guidewires in mutually different curved shapes, wherein the groups of guidewire holding surfaces are arranged for holding guidewires on mutually opposite sides of the guidewire torque, so that the guidewire holding surfaces of one group does not interfere with the guidewire holding surfaces of a group on the other side and a great freedom of design regarding the curvature imposed can be obtained in a compact torquer design.

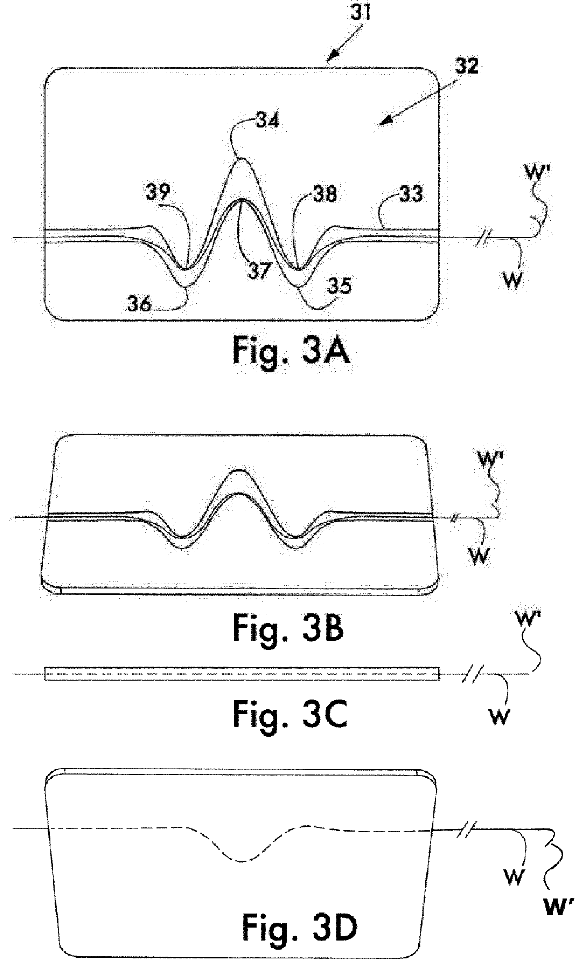

Fig. 3A shows a front view of a guidewire torquer 31. The guidewire torquer 31 is comprised of a handling body 32 and an insertion channel 33 constituted by guidewire holding surfaces and an abutment surface forming the bottom of the insertion channel. Also a guidewire W with a tip W' at its distal end is shown, resting in the insertion channel. The insertion channel 33 has a wavy or sinusoid shape having one top bend 34 and two bottom bends 35 and 36. Due to its natural stiffness the guidewire reaches the position in which the guidewire W firmly touches the inner apexes 37, 38 and 39 of the bends in the insertion channel 33.

Due to the resulting friction between the guidewire W and the guidewire torquer 31 the guidewire substantially becomes functionally one with the guidewire torquer.

The insertion channel 33 has an open side that is open to the side of the surface in which the channel 33 is arranged. Because, the open side of the channel is narrower than a largest width of the channel 33, the guidewire W is reliably prevented from flipping out of the channel 33. If the width of a passage at the open side is slightly narrower than the diameter of the guidewire W and of a stiffness allowing a guidewire W to be snapped through the passage into a position between the guidewire holding surfaces, a particularly reliable connection of the torquer to the guidewire is achieved.

Therefore, as is illustrated in the figures 3B to 3D, the rotational orientation of the guidewire in the insertion channel is precisely defined by the rotational orientation of the guidewire torquer.

Compared to figure 3A, the guidewire torquer 31 has been tilted 45 degrees backwards in figure 3B, resulting concurrently in a similar change in rotational orientation of the distal tip W' backwards over 45 degrees. Likewise, in the figures 3C and 3D a backwards tilt of the guidewire torquer over 90 and 135 degrees directly causes a corresponding backwards rotation of the distal tip W' over 90 and 135 degrees.

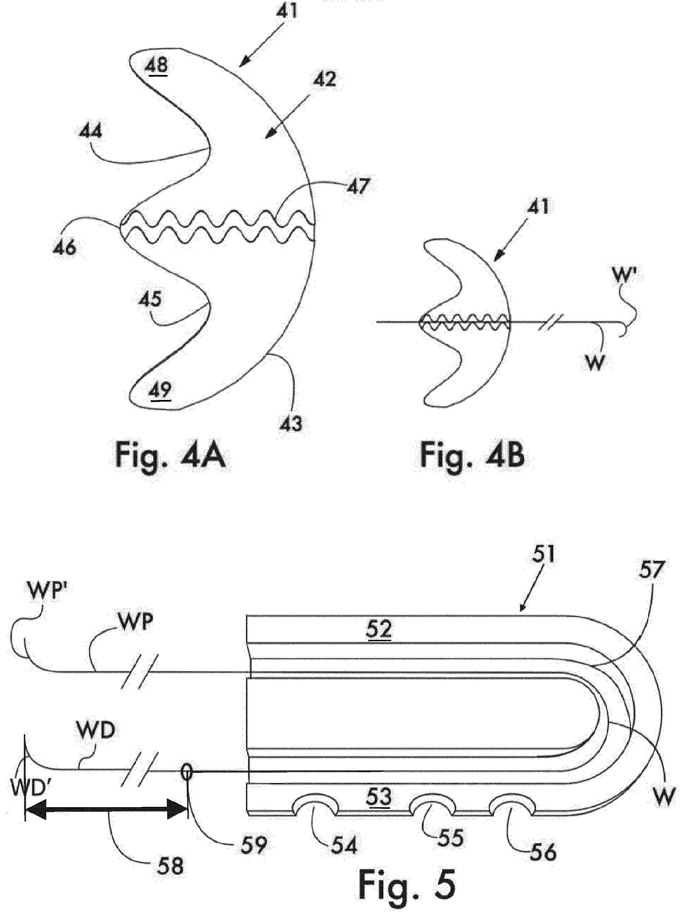

Fig. 4A shows a further preferred embodiment of a guidewire torquer 41 with a substantially flat handling body 42 in a shape having a convex right side 43 and a left side with 2 recesses 44, 45 and a protrusion 46 in between. An insertion channel 47 with multiple low amplitude bends extends from the protrusion 46 to the convex right side 43 of this embodiment. The top side 48 and the bottom side 49 of the guidewire torquer 41 are symmetrically and widely spaced from the insertion channel 47. Fig. 4B shows the guidewire torquer 41 mounted on a guidewire W whose distal tip W' has a curved shape.

Functionally, the configuration of this embodiment provides the guidewire torquer 41 with a stable position when positioned on a flat surface. A rotational force exerted to the guidewire torquer, transmitted to the torquer e.g. by the guidewire W, will not result in rotation of the guidewire torquer. Therefore, withstanding the exerted rotational force, the guidewire torquer 41 will remain unchanged in its stable position relative to the flat surface it has been laid upon. As described in the above, when a guidewire torquer of the present invention is mounted on a guidewire, together they form one functional unit due to the firm fixation of the torquer relative to the guidewire. Therefore, the embodiment shown in figure 4, as well as other embodiments, serves as a stabilizer relative to the surface it is laid upon of the rotational orientation both of the guidewire torquer 41 and of the guidewire W.

Moreover the purpose of this embodiment is to keep the tip W' of the guidewire reliably pointing to a specific desired direction during advancement of the guidewire in the human body. In fig. 4B the tip W' is directed into the same direction as the bottom side 49 of the guidewire torquer 41, which direction is downwards. Due to its substantially flat shape the guidewire torquer 41 will remain in parallel position to the surface it is laid upon when it is dragged to the right over the surface by the guidewire, in a procedure where the physician advances the tip W' of the guidewire further into the human body.

An example of such procedure is a placement of a central vein catheter (CVC). The CVC is fed over the guidewire W which tip W' first has been navigated from the subclavian vein into the central vein. During the navigation of the guidewire to the central vein the guidewire torquer 41 keeps the tip W' of the guidewire constantly directed downwards, that is into the direction of the central vein. Consequently, the tip W' preferentially enters the central vein. So, the function of the guidewire torquer 41 is to allow for the correct placement of the guidewire W in the central vein, and therefore to allow for the correct placement of the CVC that is fed over it.

By doing so the guidewire torquer 41 prevents the tip W' of the guidewire to turn upwards and erroneously enter the jugular vein, resulting in the CVC erroneously being fed over the guidewire into the jugular vein as well. Such misplacement of the CVC is the most frequent complication of CVC placement procedures.

Fig. 5 provides a preferred embodiment in which a guidewire torquer 51 has a thicker top side 52 than a bottom side 53. This asymmetry provides the user tactile information as to the position of the top side 52 relatively to the position of the bottom side 53 of the guidewire torquer 51. The bottom side 53 has three indentations 54, 55 and 56, that allow for further improved tactile feedback as well as for a firm grip on the guidewire torquer by fingers of the physician when feeling the indentations or placed therein.

The guidewire torquer 51 has an insertion channel 57 that is substantially shaped like a U-turn. Therefore, a proximal portion WP of a guidewire W that has been inserted in the U-turn shaped insertion channel 57 points into the same direction as a distal portion WD of the guidewire W. The proximal portion WP has a proximal tip WP' with a shape that resembles the shape of the distal tip WD'.

A combination of the guidewire W and the guidewire torquer 51 provides the physician with information as to both the rotational orientation and the insertion depth of the distal end WD' of the guidewire W. In the text the insertion depth of a guidewire measures a distance from a distal end of the guidewire located inside the human body to a point of entry of the guidewire into the patient. In figure 5 the insertion depth of the guidewire W measures the distance 58 between the distal end WD' and the point of entry 59 of the guidewire into the human body. Without any measurement the insertion depth of the guidewire W is visually indicated by the position of the proximal tip WP', whenever the guidewire has been positioned in the insertion channel in a symmetrical way relatively to the curve of the U-turn insertion channel, which is the case in figure 5.

Using the guidewire torquer 51 can be highly advantageous because it allows the physician to substantially reduce screening time and therefore the procedural radiation exposure. Indeed, feedback that is provided by the guidewire torquer 51 about the rotational orientation and the insertion depth of the guidewire is available for the physician without the need for additional fluoroscopy. Obtaining such information without the said feedback inevitably requires screening of the guidewire inside the patient, thereby increasing the amount of radiation exposure and prolonging the procedure time. In contrast, the rotational orientation and the insertion depth of the distal tip of the guidewire inside the human body can be easily and continuously derived from the rotational orientation and position of the proximal tip that is readily visible outside the human body.

The present invention is described in the foregoing on the basis of several preferred embodiments. Different aspects of different embodiments can be combined, wherein all combinations which can be made by a skilled person on the basis of this document must be included. These preferred embodiments are not limitative for the scope of protection of this document. The rights sought are defined in the appended claims.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A guidewire torquer for manually controlling a guidewire (W) and imparting motion to a guidewire (W), the guidewire torquer (1, 31, 41, 51) comprising:
a handling body (2, 32, 42) for handling of the guidewire torquer (1, 31, 41, 51), and
guidewire holding surfaces (7, 8) oriented for engaging the guidewire (W) from mutually opposite sides of the guidewire (W) and holding the guidewire (W) in an elastically tensioned, bent guidewire shape (3) providing frictional fixation of the guidewire (W) relative to the handling body (2, 32, 42) in a position extending along a guidewire trajectory along and touching the guidewire holding surfaces (7, 8),
wherein the guidewire holding surfaces (7, 8) project from at least one abutment surface (5) connected with the guidewire holding surfaces via angular transitions (27, 28),
wherein the guidewire trajectory is accessible from a direction in which the at least one abutment surface (5) is facing,
**characterised in that**, at least two of the guidewire holding surfaces (7, 8) lean over obliquely towards the abutment surface (5) at an angle of more than 25°, and
wherein the guidewire holding surfaces (7, 8) are part of a single, rigid body portion (2, 32, 42), at least the handling body (2, 32, 42) being of a material having a modulus of elasticity of at least 0.5 GPa.

2. A guidewire torquer according to claim 1, further comprising at least two groups of guidewire holding surfaces (7, 8), said groups being arranged for holding guidewires (W) in mutually different curved shapes, wherein said groups of guidewire holding surfaces (7, 8) are arranged for holding guidewires on mutually opposite sides of the guidewire torquer (1, 31, 41, 51).

3. A guidewire torquer according to claim 1 or 2, wherein at least portions of the guidewire holding surfaces (7, 8) have a higher coefficient of friction relative to a guidewire (W) than other surface portions of the torquer (1, 31, 41, 51).

4. A guidewire torquer according to any of the preceding claims, in which the guidewire holding surfaces (7, 8) form a curved insertion channel (33, 47, 57) in the handling body (2, 32, 42), and in which the insertion channel (33, 47, 57) has an open side that is open to the side of the surface in which the channel is arranged, wherein the insertion channel (33, 47, 57) has at least one portion in which, seen in cross-section, the open side of the channel is narrower than a largest width of the channel.

5. A guidewire torquer according to any of the preceding claims, wherein at least two of the guidewire holding surfaces (7, 8) lean over obliquely towards the abutment surface (5) at an angle of more than 30° and more preferably more than 35°.

6. A guidewire torquer according to any of the preceding claims, wherein at least the handling body (2, 32, 42) is of a material having a modulus of elasticity of at least 1.0 GPa and preferably at least 1.5 GPa.

7. A guidewire torquer according to any of the preceding claims, wherein at least the handling body (2, 32, 42) has a material thickness at the abutment surface (5) of at least 2 mm and preferably at least 3 mm.

8. A guidewire torquer according to any of the preceding claims, wherein the guidewire holding surfaces (7, 8) are arranged such that, in a view of said bent guidewire shape (3) perpendicular to a plane in which the guidewire is curved, the amplitude of a succession of bends (11-13; 34-36) is more than 0.1 and preferably more than 0.2 times the distance between successive peaks or successive deepest positions of valleys in the succession of bends (11-13; 34-36).

9. A guidewire torquer according to any of the preceding claims, wherein the guidewire holding surfaces (7, 8) are arranged such that, in a view of said bent guidewire shape (3) perpendicular to a plane in which the guidewire is curved, the succession of bends (11-13; 34-36) constitutes at least two peaks or at least two valleys.

10. A kit comprising:
a guidewire (W), and
a guidewire torquer (1) according to any of the preceding claims.

## Patentansprüche

1. Führungsdraht-Drehmomenterzeuger zum manuellen Steuern eines Führungsdrahts (W) und zum Übertragen einer Bewegung an einem Führungsdraht (W), wobei der Führungsdraht-Drehmomenterzeuger (1, 31, 41, 51) Folgendes umfasst:
einen Handhabungskörper (2, 32, 42) zur Handhabung des Führungsdraht-Drehmomenterzeugers (1, 31, 41, 51) und
Führungsdrahthalteflächen (7, 8), die so ausgerichtet sind, dass sie den Führungsdraht (W) von einander gegenüberliegenden Seiten des Führungsdrahts (W) greifen und den Führungsdraht (W) in einer elastisch gespannten, gebogenen Führungsdrahtform (3) halten, die eine reibschlüssige Fixierung des Führungsdrahts (W) relativ zu dem Handhabungskörper (2, 32, 42) in einer Position gewährleistet, die sich entlang einer Führungsdrahtbahn entlang der Führungsdrahthalteflächen (7, 8) erstreckt und diese berührt,
wobei die Führungsdrahthalteflächen (7, 8) von mindestens einer Anlagefläche (5) vorstehen, die mit den Führungsdrahthalteflächen über Winkelübergänge (27, 28) verbunden ist,
wobei die Führungsdrahtbahn aus einer Richtung zugänglich ist, in die die mindestens eine Anlagefläche (5) gerichtet ist,
**dadurch gekennzeichnet, dass**
sich mindestens zwei der Führungsdrahthalteflächen (7, 8) schräg zur Anlagefläche (5) in einem Winkel von mehr als 25° neigen und
wobei die Führungsdrahthalteflächen (7, 8) Teil eines einzigen steifen Körperabschnitts (2, 32, 42) sind, wobei mindestens der Handhabungskörper (2, 32, 42) aus einem Material mit einem Elastizitätsmodul von mindestens 0,5 GPa besteht.

2. Führungsdraht-Drehmomenterzeuger nach Anspruch 1, weiter umfassend mindestens zwei Gruppen von Führungsdrahthalteflächen (7, 8), wobei die Gruppen zum Halten von Führungsdrähten (W) in voneinander verschiedenen gekrümmten Formen angeordnet sind, wobei die Gruppen von Führungsdrahthalteflächen (7, 8) angeordnet sind, um Führungsdrähte auf einander gegenüberliegenden Seiten des Führungsdrahtmoments (1, 31, 41, 51) zu halten.

3. Führungsdraht-Drehmomenterzeuger nach Anspruch 1 oder 2, wobei mindestens Teile der Führungsdrahthalteflächen (7, 8) einen relativ zu einem Führungsdraht (W) höheren Reibungskoeffizienten aufweisen als andere Oberflächenabschnitte des Drehmomenterzeugers (1, 31, 41, 51).

4. Führungsdraht-Drehmomenterzeuger nach einem der vorhergehenden Ansprüche, bei dem die Führungsdrahthalteflächen (7, 8) einen gekrümmten Einführungskanal (33, 47, 57) im Handhabungskörper (2, 32, 42) bilden und bei dem der Einführkanal (33, 47, 57) eine offene Seite hat, die zu der Seite der Oberfläche offen ist, in der der Kanal angeordnet ist, wobei der Einführkanal (33, 47, 57) mindestens einen Abschnitt aufweist, in dem, gesehen im Querschnitt, die offene Seite des Kanals schmaler als eine größte Breite des Kanals ist.

5. Führungsdraht-Drehmomenterzeuger nach einem der vorhergehenden Ansprüche, bei dem sich mindestens zwei der Führungsdrahthalteflächen (7, 8) in einem Winkel von mehr als 30° und bevorzugter mehr als 35° zur Anlagefläche (5) hin schräg neigen.

6. Führungsdraht-Drehmomenterzeuger nach einem der vorhergehenden Ansprüche, wobei mindestens der Handhabungskörper (2, 32, 42) aus einem Material mit einem Elastizitätsmodul von mindestens 1,0 GPa und vorzugsweise mindestens 1,5 GPa besteht.

7. Führungsdraht-Drehmomenterzeuger nach einem der vorhergehenden Ansprüche, bei dem mindestens der Handhabungskörper (2, 32, 42) an der Anlagefläche (5) eine Materialstärke von mindestens 2 mm und vorzugsweise mindestens 3 mm aufweist.

8. Führungsdraht-Drehmomenterzeuger nach einem der vorhergehenden Ansprüche, wobei die Führungsdrahthalteflächen (7, 8) so angeordnet sind, dass in einer Ansicht der gebogenen Führungsdrahtform (3) senkrecht zu einer Ebene, in der der Führungsdraht gekrümmt ist, die Amplitude einer Folge von Biegungen (11-13; 34-36) mehr als das 0,1-fache und vorzugsweise mehr als das 0,2-fache des Abstandes zwischen aufeinanderfolgenden Spitzen oder aufeinanderfolgenden tiefsten Positionen von Tälern in der Folge von Biegungen (11-13; 34-36) beträgt.

9. Führungsdraht-Drehmomenterzeuger nach einem der vorhergehenden Ansprüche, wobei die Führungsdrahthalteflächen (7, 8) so angeordnet sind, dass in einer Ansicht der gebogenen Führungsdrahtform (3) senkrecht zu einer Ebene, in der der Führungsdraht gekrümmt ist, die Aufeinanderfolge der Kurven (11-13; 34-36) mindestens zwei Gipfel oder mindestens zwei Täler bildet.

10. Kit bestehend aus:
einem Führungsdraht (W) und
einem Führungsdraht-Drehmomenterzeuger (1) nach einem der vorhergehenden Ansprüche.

## Revendications

1. Dispositif de torsion de fil-guide pour commander manuellement un fil-guide (W) et imprimer un mouvement à un fil-guide (W), le dispositif de torsion de fil-guide (1, 31, 41, 51) comprenant :
un corps de manipulation (2, 32, 42) pour manipuler le dispositif de torsion de fil-guide (1, 31, 41, 51), et
des surfaces de maintien de fil-guide (7, 8) orientées pour venir en prise avec le fil-guide (W) depuis des côtés mutuellement opposés du fil-guide (W) et maintenir le fil-guide (W) dans une forme de fil-guide courbé sous tension élastique (3) fournissant une fixation par friction du fil-guide (W) par rapport au corps de manipulation (2, 32, 42) dans une position s'étendant le long d'une trajectoire de fil-guide le long des surfaces de maintien de fil-guide (7, 8) et en contact avec celles-ci,
dans lequel les surfaces de maintien de fil-guide (7, 8) font saillie à partir d'au moins une surface de butée (5) reliée aux surfaces de maintien de fil-guide via des transitions angulaires (27, 28),
dans lequel la trajectoire de fil-guide est accessible depuis une direction à laquelle la au moins une surface de butée (5) fait face,
**caractérisé en ce qu'**au moins deux des surfaces de maintien de fil-guide (7, 8) sont inclinées de manière oblique vers la surface de butée (5) selon un angle supérieur à 25°, et
dans lequel les surfaces de maintien de fil-guide (7, 8) font partie d'une partie de corps rigide unique (2, 32, 42), au moins le corps de manipulation (2, 32, 42) étant en un matériau ayant un module d'élasticité d'au moins 0,5 GPa.

2. Dispositif de torsion de fil-guide selon la revendication 1, comprenant en outre au moins deux groupes de surfaces de maintien de fil-guide (7, 8), lesdits groupes étant agencés pour maintenir des fils-guides (W) dans des formes courbes différentes les unes des autres, lesdits groupes de surfaces de maintien de fil-guide (7, 8) sont agencés pour maintenir des fils-guides sur des côtés mutuellement opposés du dispositif de torsion de fil-guide (1, 31, 41, 51).

3. Dispositif de torsion de fil-guide selon la revendication 1 ou 2, dans laquelle au moins des parties des surfaces de maintien de fil-guide (7, 8) ont un coefficient de frottement plus élevé par rapport à un fil-guide (W) que d'autres parties de surface du dispositif de torsion (1, 31, 41, 51).

4. Dispositif de torsion de fil-guide selon l'une quelconque des revendications précédentes, dans laquelle les surfaces de maintien de fil-guide (7, 8) forment un canal d'insertion courbe (33, 47, 57) dans le corps de manipulation (2, 32, 42), et dans lequel le canal d'insertion (33, 47, 57) a un côté ouvert qui est ouvert sur le côté de la surface dans laquelle le canal est agencé, dans lequel le canal d'insertion (33, 47, 57) a au moins une partie dans laquelle, vu en coupe transversale, le côté ouvert du canal est plus étroit que la plus grande largeur du canal.

5. Dispositif de torsion de fil-guide selon l'une quelconque des revendications précédentes, dans lequel au moins deux des surfaces de maintien de fil-guide (7, 8) sont inclinées de manière oblique vers la surface de butée (5) selon un angle supérieur à 30°, et de manière plus préférée supérieur à 35°.

6. Dispositif de torsion de fil-guide selon l'une quelconque des revendications précédentes, dans laquelle au moins le corps de manipulation (2, 32, 42) est en un matériau ayant un module d'élasticité d'au moins 1,0 GPa, et de préférence d'au moins 1,5 GPa.

7. Dispositif de torsion de fil-guide selon l'une quelconque des revendications précédentes, dans laquelle au moins le corps de manipulation (2, 32, 42) a une épaisseur de matériau au niveau de la surface de butée (5) d'au moins 2 mm, et de préférence d'au moins 3 mm.

8. Dispositif de torsion de fil-guide selon l'une quelconque des revendications précédentes, dans laquelle les surfaces de maintien de fil-guide (7, 8) sont agencées de telle sorte que, dans une vue de ladite forme de fil-guide courbe (3) perpendiculaire à un plan dans lequel le fil-guide est courbé, l'amplitude d'une succession de courbes (11-13 ; 34-36) est supérieure à 0,1 et de préférence supérieure à 0,2 fois la distance entre des pics successifs ou des positions les plus profondes successives de vallées dans la succession de courbes (11-13 ; 34-36).

9. Dispositif de torsion de fil-guide selon l'une quelconque des revendications précédentes, dans laquelle les surfaces de maintien de fil-guide (7, 8) sont agencées de telle sorte que, dans une vue de ladite forme de fil-guide courbe (3) perpendiculaire à un plan dans lequel le fil-guide est courbé, la succession de courbes (11-13 ; 34-36) constitue au moins deux pics ou au moins deux vallées.

10. Kit, comprenant :
un fil-guide (W), et
un dispositif de torsion de fil-guide (1) selon l'une quelconque des revendications précédentes.
